# EUROPEAN PATENT APPLICATION

(11) **EP 3 587 581 A1**
(43) Date of publication of application: **01.01.2020**
(21) Application number: 18179977.6
(22) Date of filing: 26.06.2018
(51) Int. Cl.: C12N 15/86

(54) **FORMULATIONS FOR SIMIAN ADENOVIRAL VECTORS HAVING ENHANCED STORAGE STABILITY**

(71) Applicant: GlaxoSmithKline Biologicals S.A., 1330 Rixensart (BE)
(72) Inventor: The designation of the inventor has not yet been filed
(74) Representative: Evans, Stephen John Eves

(57) **Abstract**

The invention relates to liquid formulations of simian adenoviruses and methods for obtaining the formulations. The formulations comprise an amorphous sugar, a surfactant, a bivalent metal salt and an alpha tocopherol in a buffered solution.

## Description

### FIELD OF THE INVENTION

The invention relates to the formulation of adenoviral vectors in liquid compositions, their formulations and methods of using the compositions.

### BACKGROUND

Adenoviral vectors represent a prophylactic or therapeutic protein delivery platform whereby the nucleic acid sequence encoding the prophylactic or therapeutic protein is incorporated into the adenoviral genome, which is brought to expression when the adenoviral particle is administered to the treated subject. It has been a challenge in the art to develop stabilizing formulations for the adenoviral vectors which allow storage at acceptable storage temperatures with a considerable shelf life.

Stabilizing liquid formulations have been reported for human adenoviral vectors such as described by R.K Evans et al. (Development of stable liquid formulations for adenovirus-based vaccines, J. Pharm. Sci. (2004) 93:2458-2475). Stabilizing simian adenovirus by lyophilization has also been reported (WO 2017/013169; Mathot et al., BioProcess Int. (2018)). However, there remains a need in the art for formulations that preserve the stability of simian adenoviral vectors in order to decrease vaccine waste due to disruption of the cold chain and to facilitate vaccine production, shipment, storage and patient compliance.

### SUMMARY OF THE INVENTION

The inventors surprisingly found that tocopherol and recombinant human serum albumin greatly increased the stability of simian adenovirus when incorporated into a composition comprising amorphous sugar. The invention therefore provides a stable aqueous liquid formulation for simian adenoviruses comprising tocopherol, recombinant human serum albumin and at least one amorphous sugar. Adenoviruses formulated as described herein are thermostable. The invention further provides methods of using the stabilized recombinant simian adenoviruses to confer prophylactic immunity and to act as therapeutic vectors by delivering a transgene to a human subject.

### BRIEF DESCRIPTION OF THE DRAWINGS

FIG. 1: Effect of osmolality and sugar composition on stability. Stability was measured by PICOGREEN following exposure of the virus to a temperature of 4°C for three months or 30°C for one month.
FIG. 2: Effect of trehalose, sucrose, VES and rHSA on stability. "BDSADVAR" is in 10 mM Tris pH 8.5, 5 mM NaCl, 10 mM histidine, 0.025% polysorbate 80 (w/v) and 1 mM MgCl_{2.} Stability was measured by analytical HPLC after exposure of the virus to 4°C or 25°C for three weeks.
FIG. 3A: Real time stability at 4°C. Adenovirus in 10 mM Tris pH 8.5, 5 mM NaCl, 10 mM histidine, 0.024% polysorbate 80 (w/v), 1 mM MgCl₂, 15% trehalose (w/v), 2% sucrose (w/v), 0.05 mM VES and 0.1% rHSA (w/v) at a concentration (comprising a 10% overage) of 1.1 x 10¹¹ PU/ml (virus particle units per milliliter) over a six-month period measured by two independent analytical HPLC methods (solid and dotted lines).
FIG. 3B: Real time stability at 4°C. Adenovirus in 10 mM Tris pH 8.5, 5 mM NaCl, 10 mM histidine, 0.024% polysorbate 80 (w/v), 1 mM MgCl₂, 16% sucrose and 0.05mM VES at a concentration (comprising a 10% overage) of 1.1 x 10¹¹ PU/ml (virus particle units per milliliter) over a six-month period measured by two independent analytical HPLC methods (solid and dotted lines).
FIG. 3C: Real time stability at 4°C. Adenovirus in 10 mM Tris pH 8.5, 5 mM NaCl, 10 mM histidine, 0.024% polysorbate 80 (w/v), 1 mM MgCl₂, 15% trehalose (w/v), 2% sucrose (w/v), 0.05 mM VES and 0.1% rHSA (w/v) at a concentration (comprising a 10% overage) of 1.1 x 10¹⁰ PU/ml (virus particle units per milliliter) over a six-month period measured by two independent analytical HPLC methods (solid and dotted lines).
FIG. 4: Immunogenicity in mice determined by IFN gamma ELIspot and compared to lyophilized simian adenovirus..
FIG. 5: Forced degradation of simian adenovirus via metal and light oxidation. Stability was measured by analytical high performance liquid chromatography (HPLC) after exposure of the virus to metal and light oxidation under Acceleration Oxidation Test (AOT) conditions.

### DETAILED DESCRIPTION

Contrary to reports in the art on the formulation of human adenoviral vectors, the inventors found that the stabilizing formulations developed for human adenoviral vectors could not successfully be applied to all adenoviral vectors, e.g. simian adenoviral vectors for prophylaxis or therapy. For example, A195 buffer (10 mM Tris pH7.4, 10 mM histidine, 75 mM NaCl, 5% sucrose, 0.02% polysorbate 80, 0.1 mM EDTA, 1 mM MgCl₂), which is used to stabilize human adenovirus, is not suitable for stabilizing simian adenoviruses (Mathot et al., BioProcess Int. (2018)). The present invention describes compositions of simian adenovirus wherein the structural integrity and functionality of the adenoviral particle is better protected or maintained.

The novel formulations of the invention allow storage of an aqueous liquid composition comprising simian adenovirus at 4°C for up to two years and at 25°C for up to two weeks. Under these conditions, a simian adenovirus vaccine is expected to lose about 50% of its infectivity, which is considered acceptable in the vaccine field.

### Simian Adenoviruses

Adenoviruses are nonenveloped viruses with an icosahedral capsid that contains a double stranded DNA genome. The capsid comprises three major proteins, hexon (II), penton base (III) and a knobbed fiber (IV), along with a number of other minor proteins, VI, VIII, IX, IIIa and IVa2 and mediates the early stages of adenoviral infection. The hexon accounts for the majority of the structural components of the capsid, which consists of 240 trimeric hexon capsomeres and 12 penton bases. The hexon has three conserved double barrels, while the top has three towers, each tower containing a loop from each subunit that forms most of the capsid. The base of the hexon is highly conserved between adenoviral serotypes, while the surface loops are variable. The penton is another adenoviral capsid protein that forms a pentameric base to which the fiber attaches. The trimeric fiber protein protrudes from the penton base at each of the 12 vertices of the capsid and is a knobbed rod-like structure. The primary role of the fiber protein is the tethering of the viral capsid to the cell surface via the interaction of the knob region with a cellular receptor, and variations in the flexible shaft as well as knob regions of fiber are characteristic of the different serotypes.

By "simian" is meant any member of the infraorder Simiiformes. It includes Platyrrhini (New World monkeys) and Catarrhini (Old World monkeys and apes). It includes bonobos, capuchins, chimpanzees, gibbons, gorillas, great apes, howler monkeys, marmosets, orang-utans, owl monkeys, sakis, spider monkeys, squirrel monkeys, tamarinds, titis, uakaris and woolly monkeys. Numerous adenoviruses have been isolated from simians such as chimpanzees, bonobos, rhesus macaques and gorillas. Vectors derived from these adenoviruses have been shown to induce strong immune responses to encoded transgenes (Colloca et al. (2012) Sci. Transl. Med. 4:1-9; Roy et al. (2004) Virol.324: 361-372; Roy et al. (2010) J. of Gene Med. 13:17-25). Also, simian adenoviruses demonstrate a relative lack of cross-neutralizing antibodies compared to human adenoviruses in the human population.

Adenoviruses can be used as vectors to deliver desired RNA or protein sequences, for example heterologous sequences, for *in vivo* expression. An adenoviral vector may include any genetic element including naked DNA, a phage, transposon, cosmid, episome, plasmid, or virus. Such vectors contain DNA of the simian adenovirus and an expression cassette. By "expression cassette" (or "minigene") is meant the combination of a selected heterologous gene ("transgene" or "gene of interest") and other regulatory elements necessary to drive translation, transcription and/or expression of the gene product in a host cell. In embodiments of the invention, the simian adenoviral vector may comprise one or more of a promoter, an enhancer, and a reporter gene.

In addition to the transgene, the expression cassette also may include conventional control elements which are operably linked to the transgene in a manner that permits its transcription, translation and/or expression in a cell transfected with the adenoviral vector. As used herein, "operably linked" sequences include both expression control sequences that are contiguous with the gene of interest and expression control sequences that act in trans or at a distance to control the gene of interest.

Regulatory elements, i.e., expression control sequences, include appropriate transcription initiation, termination, promoter and enhancer sequences; efficient RNA processing signals such as splicing and polyadenylation (poly A) signals including rabbit beta-globin polyA; tetracycline regulatable systems, microRNAs, posttranscriptional regulatory elements e.g., WPRE, posttranscriptional regulatory element of woodchuck hepatitis virus); sequences that stabilize cytoplasmic mRNA; sequences that enhance translation efficiency (*e.g*., Kozak consensus sequence); sequences that enhance protein stability; and when desired, sequences that enhance secretion of an encoded product.

A "promoter" is a nucleotide sequence that permits the binding of RNA polymerase and directs the transcription of a gene. Typically, a promoter is located in a non-coding region of a gene, proximal to the transcriptional start site. Sequence elements within promoters that function in the initiation of transcription are often characterized by consensus nucleotide sequences. Examples of promoters include, but are not limited to, promoters from bacteria, yeast, plants, viruses, and mammals, including simians and humans. A great number of expression control sequences, including promoters which are internal, native, constitutive, inducible and/or tissue-specific, are known in the art and may be utilized.

A "posttranscriptional regulatory element," as used herein, is a DNA sequence that, when transcribed, enhances the expression of the transgene(s) or fragments thereof that are delivered by viral vectors of the invention. Posttranscriptional regulatory elements include, but are not limited to the Hepatitis B Virus Posttranscriptional Regulatory Element (HPRE) and the Woodchuck Hepatitis Posttranscriptional Regulatory Element (WPRE). The WPRE is a tripartite cis-acting element that has been demonstrated to enhance transgene expression driven by certain, but not all promoters.

The transgene encoded by the adenoviral vector will typically encode a product useful in biology or medicine, such as a therapeutic or immunogenic protein, an enzyme, or an RNA. Desirable RNA molecules include tRNA, dsRNA, ribosomal RNA, catalytic RNAs, RNA aptamers, and antisense RNAs. One example of a useful RNA sequence is a sequence which extinguishes expression of a targeted nucleic acid sequence in the treated subject. The transgene may encode a polypeptide or protein used for treatment, *e.g.,* of genetic deficiencies, as a cancer therapeutic or vaccine, for induction of an immune response, and/or for prophylactic vaccine purposes. Particularly the polypeptide or protein is an antigen.

As used herein, induction of an immune response refers to the ability of a protein, also known as an "antigen" or "immunogen," to induce a T cell and/or a humoral immune response to the protein. Unless otherwise indicated, "therapy" or "therapeutic" may relate to either or both preventive and curative therapy.

The transgene may be used for prophylaxis or treatment, *e.g.,* as a vaccine for inducing an immune response, to correct genetic deficiencies by correcting or replacing a defective or missing gene, or as a cancer therapeutic. Particularly, the immune response is a protective immune response.

Compositions of the invention may be immunogenic compositions. Optionally, a mixture or composition of the invention may be formulated to contain other components, including, *e.g.,* further immunogen(s), *e.g.* polypeptide antigen(s), and/or adjuvants. In an embodiment, the invention provides a vaccine comprising an adjuvant. Such an adjuvant can be administered with a priming DNA vaccine encoding an antigen to enhance the antigen-specific immune response compared with the immune response generated upon priming with a DNA vaccine encoding the antigen only. Alternatively, such an adjuvant can be administered with a polypeptide antigen which is administered in an administration regimen involving the adenoviral vectors of the invention.

The immune response elicited by the transgene may be an antigen specific B cell response, which produces neutralizing antibodies. The elicited immune response may be an antigen specific T cell response, which may be a systemic and/or a local response. The antigen specific T cell response may comprise a CD4+ T cell response, such as a response involving CD4+ T cells expressing cytokines, *e.g.* interferon gamma (IFN gamma), tumor necrosis factor alpha (TNF alpha) and/or interleukin 2 (IL2). Alternatively, or additionally, the antigen specific T cell response comprises a CD8+ T cell response, such as a response involving CD8+ T cells expressing cytokines, *e.g.,* IFN gamma, TNF alpha and/or IL2.

Thus, a composition of the invention is for use in prophylactic (*i.e.,* immunogenic or preventive) or therapeutic treatment of a subject, as a result of the action of the transgene encoded by the adenoviral vector. Compositions of the invention are suitable for intramuscular injection.

The methods of the invention induce a protective immune response, *i.e.,* by immunizing or vaccinating a subject against a pathogen. The invention may therefore be applied for the prophylaxis, treatment or amelioration of diseases due to infection by pathogens, *e.g.,* bacteria, fungi, parasitic microorganisms or multicellular parasites which infect human and non-human vertebrates, or from a cancer cell or tumor cell. Immunogens may be selected from a variety of viruses, bacteria and fungi.

A "thermostable" adenovirus formulation is one in which the adenovirus can resist irreversible change in its physical or chemical structure without losing its characteristic properties at moderately high relative temperatures.

The term "replication-defective " or "replication-incompetent" adenovirus refers to an adenovirus that is incapable of replication because it has been engineered to comprise at least a functional deletion (or "loss-of-function" mutation), *i.e.* a deletion or mutation which impairs the function of a gene without removing it entirely, *e.g.* introduction of artificial stop codons, deletion or mutation of active sites or interaction domains, mutation or deletion of a regulatory sequence of a gene etc., or a complete removal of a gene encoding a gene product that is essential for viral replication, such as one or more of the adenoviral genes selected from E1A, E1B, E2A, E2B, E3 and E4 (such as E3 ORF1, E3 ORF2, E3 ORF3, E3 ORF4, E3 ORF5, E3 ORF6, E3 ORF7, E3 ORF8, E3 ORF9, E4 ORF7, E4 ORF6, E4 ORF4, E4 ORF3, E4 ORF2 and/or E4 ORF1). Suitably, E1 and optionally E3 and/or E4 are deleted. If deleted, the aforementioned deleted gene region will suitably not be considered in the alignment when determining percent identity with respect to another sequence.

The term "replication-competent" adenovirus refers to an adenovirus which can replicate in a host cell in the absence of any recombinant helper proteins comprised in the cell. Suitably, a "replication-competent" adenovirus comprises intact structural genes and the following intact or functionally essential early genes: E1A, E1B, E2A, E2B and E4.

In an embodiment of the invention, the vector is a functional or an immunogenic derivative of an adenoviral vector. By "derivative of an adenoviral vector" is meant a modified version of the vector, *e.g.,* one or more nucleotides of the vector are deleted, inserted, modified or substituted.

Simian adenoviruses of the invention can be generated using techniques known to those of skill in the art. They can be produced in any suitable cell line in which the virus is capable of replication. Replication defective viruses can be produced in complementing cell lines which provide the factors missing from the viral vector that result in its impaired replication characteristics (such as E1).

Vectors of the invention are generated using techniques and sequences provided herein, in conjunction with techniques known to those of skill in the art. Such techniques include conventional cloning techniques of cDNA such as those described in texts, use of overlapping oligonucleotide sequences of the adenovirus genomes, polymerase chain reaction, and any suitable method which provides the desired nucleotide sequence.

### Excipients

Excipients of the invention can include buffers, salts, surfactants, sugars, organic compounds and proteins. Excipients of the invention act to stabilize the simian adenovirus while it is in an aqueous formulation.

"Buffer" refers to a substance capable of neutralizing both an acid and a base, thereby maintaining the pH of a solution. Suitable buffers of the invention include Tris, succinate, borate, Tris-maleate, lysine, histidine, glycine, glycylglycine, citrate, carbonate or combinations thereof.

"Salt" refers to ionic compounds that result from the neutralization reaction of an acid and a base, composed of a related number of cations and anions such that the product is without net charge, for example sodium chloride. The component ions can be either inorganic or organic, and can be monoatomic or polyatomic. The metallic salt MgCl₂ can be used to stabilize simian adenoviruses, as demonstrated herein by PICOGREEN assay and infectivity testing following thermal stress tests.

Excipients of the invention can include a surfactant selected from polysorbate surfactants (*e.g.* polysorbate 80 and/or polysorbate 20), poloxamer surfactants (*e.g.* poloxamer 188), octoxinal surfactants, polidocanol surfactants, polyoxyl stearate surfactants, polyoxyl castor oil surfactants, N-octyl-glucoside surfactants, macrogol 15 hydroxy stearate, and combinations thereof. In an embodiment, the surfactant is selected from poloxamer surfactants (*e.g.* poloxamer 188), polysorbate surfactants (*e.g.* polysorbate 80 and/or polysorbate 20), in particular polysorbate surfactants such as polysorbate 80. Surfactants can also be used to formulate tocopherols.

"Vitamin E," i.*e*., tocopherol, refers to a series of chiral organic molecules that vary in their degree of methylation of the phenol moiety of the chromanol ring. Tocopherols act as lipid soluble anti-oxidants.

The term includes alpha, beta, gamma and delta tocopherols. Various tocopherol salts include tocopherol succinate, tocopherol acetate, tocopherol nicotinate and other esterified forms.

By "vitamin E succinate" or "VES" is meant any alpha tocopherol succinate, including but not limited to 4-oxo-4-[[2,5,7,8-tetramethyl-2-(4,8,12-trimethyltridecyl)-3,4-dihydrochromen-6-yl]oxy]butanoic acid, D-alpha-tocopherol succinate, semisynthetic D-alpha-tocopherol succinate, alpha tocopherol acid succinate, RRR-alpha-tocopherol hydrogen succinate, D-alpha- tocopherol succinate, (+)-alpha-tocopherols, (+)-delta tocopherols, tocopherol hemisuccinate and tocopheryl acid succinate. Typically the empirical formula will be C₃₃H₅₄O₅ (Hill notation). VES can be solubilized in ethanol and may be present in residual amounts in a buffer comprising VES.

By "recombinant human serum albumin," "rHSA" or "human albumin" is meant a protein encoded by the human ALB gene and produced by recombination methods known in the art.

TRAVASOL is a solution of essential and nonessential amino acids comprising leucine, isoleucine, lysine, valine, phenylalanine, histidine, threonine, methionine, tryptophan, alanine, arginine, glycine, proline, serine and tyrosine with acetate and chloride anions at pH 5.0-7.0.

Adenovirus is stable when frozen but, unless formulated appropriately, rapidly degrades at warmer temperatures. Degradation can be caused by factors such as, but not limited to, heat; oxidation, *e.g.,* due to light, peroxide or metals; shear stress; the impact of an air interface on a liquid formulation; freeze thaw cycles; or combinations of these factors. The pathways by which adenoviruses undergo degradation include capsid disruption, aggregation, oxidation and deamidation.

"Stability" refers to resistance to degradation. Adenoviral stability can be measured by the integrity of the viral capsid. For example, the PICOGREEN assay utilizes a fluorescent nucleic acid stain that is selective for double stranded DNA. HPLC can quantify intact viral particles. The percentage of released DNA is based on a regression curve comprising a freshly thawed control group and a completely degraded control.

Quantitative PCR can quantify viral DNA (gE/ml = genome equivalents per ml.) Stability can also be determined by measuring the infectivity of the virus, *e.g.,* retention of infectivity upon storage.

"Infectivity" refers to the ability of a vector to enter into a susceptible host, *i.e.* a cell, and deliver its genetic material for expression by the host. Infectivity can be determined by measuring the entry of viral particles into cells, for example by immunostaining a viral protein. Infectivity assays are known in the art and include "cell culture infectious dose 50% (CCID₅₀)", single "infectious unit (IFU)" plaque assay and FACS analysis of cells positive for Matrix protein ("Protein M") (J. Virol. 82:8863 (2008)); or hexon protein immunostaining.

Infectivity can be determined by measuring the proportion of cells that express a transgene. For example, green fluorescent protein can be used as an infectivity marker whereby the number of cells expressing green fluorescent protein after incubation with the vector is measured.

The CCID₅₀ is the infectious dose that will infect 50% of the cells challenged with the defined inoculum and can be used to measure viral amplification and cell re-infection. Measuring the CCID₅₀ measures viral entry into the cells and viral replication. It does not measure the number of virus particles. The read-out for the CCID₅₀ is immunostaining of the hexon protein, determined by microscopy. The quantification is based on the amount of virus required to infect 50% of the cultured cells and can be expressed as log CCID₅₀/ml.

The IFU is a measure of the number of infectious virus particles, *e.g.,* per ml or per dose, and provides a measure of viral entry in to the cell and replication. IFU is a plaque-based assay, the read-out is immunostaining of the hexon protein, determined by microscopy and can be expressed as infectious units per milliliter (IFU/ml). The quantification is based on the number of infective particles, with each plaque representing one infective particle.

Infectivity by hexon or Protein M refers to the ability to infect cells at a given time point and can be used to measure viral replication. The read-out is immunostaining of Protein M or the hexon protein, determined by FACS analysis. The quantification is based on the number of positive cells.

The aqueous compositions of the invention may be contained in a glass vial, which may be either siliconized or non-siliconized. They may be contained in prefilled syringes, plastic containers with or without a blow-fill-seal, microneedle devices for intra-dermal administration and other containers used for containing viruses.

### Embodiments of the Invention

In specific embodiments, the adenoviral vector is derived from a simian adenovirus. For example, the simian adenovirus may be an adenovirus from a New World monkey, an Old World monkey or an ape. It may be an adenovirus from a chimpanzee, a bonobo, a gorilla or a macaque. Chimpanzee adenoviruses of the invention include, but are not limited to ChAd3, ChAd63, ChAd83, ChAd155 and ChAd157. Examples of such strains are described in WO03/000283, WO2010/086189 and WO 2016/198621. Bonobo adenoviruses of the invention include but are not limited to PanAd1, PanAd2 or PanAd3, Pan 5, Pan 6, Pan 7 (also referred to as C7) and Pan 9. Bonobo vectors can be found, for example, in WO2005/071093 and WO2010/086189. Adenoviral vectors may also be derived from adenoviruses isolated from gorillas as described in WO2013/52799, WO2013/52811 and WO2013/52832.

Compositions of the invention comprise a buffer selected from Tris, succinate, borate, Tris-maleate, lysine, histidine, glycine, glycylglycine, citrate, carbonate or combinations thereof. In one embodiment, the buffer is Tris, succinate or borate. In a further embodiment, the buffer is Tris. The buffer can be present in an amount of at least 1 mM, at least 2.5 mM or at least 5 mM. The buffer can be present in an amount of less than 25 mM, less than 20 mM or less than 15 mM. For example, the buffer can be present in an amount of 1 to 25 mM, 2.5 to 20 mM or 5 to 15 mM. In an embodiment, the buffer is present in an amount of about 10 mM.

In an embodiment, compositions of the invention also comprise histidine in an amount of up to about 15 mM, for example at a concentration of about 10 mM.

In an embodiment, compositions of the invention also comprise NaCl in an amount of up to about 20 mM, for example, at a concentration of about 5 mM.

In an embodiment, compositions of the invention also comprise bivalent metal ions, such as Mg²⁺ or Ca²⁺ or bivalent metal ions in the form of a salt, such as MgCl₂, CaCl₂ or MgSO₄. In an embodiment the bivalent metal ion is Mg²⁺. The bivalent metal ions can be present in an amount of about 0.1 to 10 mM or about 0.5 to 5 mM. In an embodiment, the bivalent metal ion is MgCl₂ and is present in an amount of about 1 mM.

In an embodiment, compositions of the invention also comprise a poloxamer surfactant or a polysorbate surfactant. In an embodiment, the poloxamer surfactant is poloxamer 188. In an embodiment, the polysorbate surfactant is polysorbate 80 and/or polysorbate 20. The surfactant can be present in an amount of about 0.01 to 0.05% (w/v), such as about 0.02%, 0.025%, 0.03%, 0.035%, 0.04% or 0.045%. In an embodiment, the surfactant is polysorbate 80 and is present in an amount of about 0.025% (w/v).

In an embodiment, compositions of the invention also comprise an alpha tocopherol succinate. In an embodiment, the alpha tocopherol succinate is Vitamin E succinate. The alpha tocopherol succinate can be present in an amount up to about 0.5 mM, such as about 0.01 to 0.25 mM, such as 0.05mM, 0.10mM, 0.15mM or 0.20mM. In an embodiment, the alpha tocopherol succinate is Vitamin E succinate and is present in an amount of about 0.05 mM.

In an embodiment, compositions of the invention also comprise recombinant human serum albumin in an amount up to about 1% (w/v), for example in an amount of about 0.1%, 0.2%, 0.3%, 0.4%, 0.5%, 0.6%, 0.7%, 0.8% or about 0.9%.

In an embodiment, compositions of the invention comprise at least one amorphous sugar, such as sucrose, trehalose, mannose, mannitol, raffinose, lactitol, sorbitol and lactobionic acid, glucose, maltulose, iso-maltulose, lactulose, maltose, lactose, isomaltose, maltitol, palatinit, stachyose, melezitose, dextran, or a combination thereof. In an embodiment, the amorphous sugar is trehalose, sucrose or combination of sucrose and trehalose. In an embodiment, the amorphous sugar is sucrose. In an embodiment, the amorphous sugar is a combination of sucrose and trehalose. In an embodiment, the amorphous sugar is trehalose.

In an embodiment, the amorphous sugar is present in an amount of about 0% to 30% (w/v). In an embodiment, the amorphous sugar is sucrose in an amount up to about 30% (w/v). In an embodiment, sucrose is present in an amount of about 0 to 10% or 0 to 5% (w/v), for example in an amount of about 2%. In another embodiment, sucrose is present in an amount of about 10 to 30% (w/v), for example in an amount of about 16%.

In an embodiment, the amorphous sugar is trehalose in an amount up to about 30% (w/v). In an embodiment, trehalose is present in an amount of about 10 to 20% (w/v), for example in an amount of about 15%.

In an embodiment, the amorphous sugar is a combination of sucrose and trehalose in an amount of about 0 to 10% sucrose (w/v) and about 10 to 30% trehalose (w/v); in an amount of about 0 to 5% sucrose and 10 to 20% trehalose, for example in an amount of about 2% sucrose and 15% trehalose.

In a particular embodiment, compositions of the invention comprise about 1 to 25 mM Tris pH 6-10, about 0 to 15 mM histidine, about 0 to 50 mM NaCl, about 0.1 to 10 mM MgCl₂, about 0.01 to 0.05% polysorbate 80 (w/v), about 0 to 0.5 mM Vitamin E succinate, about 0 to 1% (w/v)recombinant human serum albumin and about 0 to 30% (w/v) trehalose.

In a particular embodiment, compositions of the invention comprise about 1 to 25 mM Tris pH 6-10, about 0 to 15 mM histidine, about 0 to 50 mM NaCl, about 0.1 to 10 mM MgCl₂, about 0.01 to 0.05% (w/v) polysorbate 80, about 0 to 0.5 mM Vitamin E succinate, about 0 to 1% (w/v) recombinant human serum albumin, about 0 to 10% (w/v) sucrose and about 5 to 25% (w/v) trehalose.

In a more particular embodiment, compositions of the invention comprise about 2.5 to 20 mM Tris pH 7.5 to 9.5, about 5 to 15 mM histidine, about 0 to 10 mM NaCl, about 0.5 to 5 mM MgCl₂, about 0.01 to 0.05% (w/v) polysorbate 80 w/v, about 0.01 to 0.25 mM Vitamin E succinate, about 0 to 1% w(w/v) recombinant human serum albumin and about 5 to 25% (w/v) trehalose.

In another more particular embodiment, compositions of the invention comprise about 2.5 to 20 mM Tris pH 7.5 to 9.5, about 5 to 15 mM histidine, about 0 to 10 mM NaCl, about 0.5 to 5 mM MgCl₂, about 0.01 to 0.05%(w/v) polysorbate 80, about 0.01 to 0.25 mM Vitamin E succinate, about 0 to 1% (w/v) recombinant human serum albumin and about 10 to 20% (w/v) sucrose.

In a further more particular embodiment, compositions of the invention comprise about 5 to 15 mM Tris pH 7.5 to 9.5, about 8 to 12 mM histidine, about 0.5 to 2.5 mM NaCl, about 0.5 to 5 mM MgCl₂, about 0.015 to 0.035% (w/v) polysorbate 80, about 0.025 to 0.1 mM Vitamin E succinate, about 0.1 to 0.5% (w/v) recombinant human serum albumin and about 0 to 5% (w/v) sucrose.

In a yet further more particular embodiment, compositions of the invention comprise about 5 to 15 mM Tris pH 7.5 to 9.5, about 8 to 12 mM histidine, about 0.5 to 2.5 mM NaCl, about 0.5 to 5 mM MgCl₂, about 0.015 to 0.035% (w/v) polysorbate 80, about 0.025 to 0.1 mM Vitamin E succinate, about 0.1 to 0.5% (w/v) recombinant human serum albumin, about 0 to 5% (w/v) sucrose and about 10 to 20% (w/v) trehalose.

In a yet further more particular embodiment, compositions of the invention comprise about 5 to 15 mM Tris pH 7.5 to 9.5, about 8 to 12 mM histidine, about 0.5 to 2.5 mM NaCl, about 0.5 to 5 mM MgCl₂, about 0.015 to 0.035% (w/v) polysorbate 80, about 0.025 to 0.1 mM Vitamin E succinate, about 0.1 to 0.5% (w/v) recombinant human serum albumin and about 10 to 20% (w/v) sucrose.

In a most particular embodiment, compositions of the invention comprise about 10 mM Tris pH 8.5, about 10 mM histidine, about 5 mM NaCl, about 1 mM MgCl₂, about 0.024% (w/v) polysorbate 80, about 0.05 mM Vitamin E succinate, about 0.1% (w/v) recombinant human serum albumin and about 16% (w/v) trehalose.

In another most particular embodiment, compositions of the invention comprise about 10 mM Tris pH 8.5, about 10 mM histidine, about 5 mM NaCl, about 1 mM MgCl₂, about 0.024% w(w/v) polysorbate 80, about 0.05 mM Vitamin E succinate, about 0.1% (w/v) recombinant human serum albumin, about 2% (w/v) sucrose and about 15% (w/v) trehalose.

In a further most particular embodiment, compositions of the invention comprise about 10 mM Tris pH 8.5, about 10 mM histidine, about 5 mM NaCl, about 1 mM MgCl₂, about 0.024% (w/v) polysorbate 80, about 0.05 mM Vitamin E succinate, about 0.1% (w/v) recombinant human serum albumin, and about 16% (w/v) sucrose.

In an embodiment, the invention provides a method of making an aqueous liquid composition described herein by producing a simian adenovirus in a host cell and combining the adenovirus with one or more excipients, wherein the adenovirus is stable at +2 - +8°C for at least two years.

In another embodiment, the invention provides a method of making an aqueous liquid composition described herein by producing a simian adenovirus in a host cell and combining the adenovirus with one or more excipients, wherein the adenovirus is stable at 25°C for at least 30 days.

In a further embodiment, the invention provides a method of making an aqueous liquid composition described herein by producing a simian adenovirus in a host cell and combining the adenovirus with one or more excipients, wherein the adenovirus is stable at 30°C for at least seven days.

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. Numerical limitations given with respect to concentrations or levels of a substance, such as solution component concentrations or ratios thereof, and reaction conditions such as temperatures, pressures and cycle times are intended to be approximate. The term "about" used herein is intended to mean the amount ± 10%.

The term "comprises" means "includes." Thus, unless the context requires otherwise, the word "comprises," and variations such as "comprise" and "comprising," are understood to imply the inclusion of a stated compound or composition (*e.g.,* nucleic acid, polypeptide, antigen) or step, or group of compounds or steps, but no to the exclusion of other compounds, compositions, steps or groups thereof. The abbreviation, "*e.g.*" is derived from the Latin *exempli gratia,* and is used herein to indicate a non-limiting example. Thus, the abbreviation "*e.g.*" is synonymous with the term "for example."

The present invention will now be further described by means of the following non-limiting examples.

### EXAMPLES

### Example 1: Factorial Testing of the Effects of Excipients on Stability

ChAd155-RSV (WO 2016/198599) was formulated at a concentration of 1 x 10¹¹ virus particle units ("PU/ml") in 10 mM Tris pH 8.5, 25 mM NaCl, 8% sucrose (w/v), 0.02% polysorbate 80 (w/v) and 1 mM MgCl₂, then tested for stability after being exposed to temperatures of 30°C for three days or seven days. Five excipients were tested in a 2⁵⁻¹ fractional factorial design study having five factors (the excipients) with 16 runs in the corners and four center points. The factors were (1) MgSO₄ at a concentration of 15 mM or 30 mM; (2) ethanol at a concentration of 1.54% or 3.07%; (3) TRAVASOL at a concentration of 1 mM or 4 mM; (4) rHSA at a concentration of 0.05% or 0.5%; and (5) Vitamin E succinate (VES) at a concentration of 1 mM or 2 mM. Stability was determined by measuring viral particles by analytical HPLC. The ability of the virus to retain infectivity was measured as the number of cells expressing the adenovirus hexon capsid protein after incubation with the virus and the comparisons are shown in the tables below.

| **Run** | **MgSO₄** | **EtOH** | **TRAVASOL** | **rHSA** | **VES** |
|---|---|---|---|---|---|
| 1 | 1 | -1 | 1 | -1 | 1 |
| 2 | 0 | 0 | 0 | 0 | 0 |
| 3 | 1 | -1 | -1 | -1 | -1 |
| 4 | 1 | -1 | -1 | 1 | 1 |
| 5 | 1 | 1 | 1 | 1 | 1 |
| 6 | -1 | 1 | 1 | -1 | 1 |
| 7 | 0 | 0 | 0 | 0 | 0 |
| 8 | -1 | 1 | -1 | -1 | -1 |
| 9 | 1 | 1 | 1 | -1 | -1 |
| 10 | -1 | -1 | -1 | 1 | -1 |
| 11 | -1 | -1 | 1 | -1 | -1 |
| 12 | -1 | -1 | 1 | 1 | 1 |
| 13 | 0 | 0 | 0 | 0 | 0 |
| 14 | 1 | 1 | -1 | -1 | 1 |
| 15 | -1 | -1 | -1 | -1 | 1 |
| 16 | -1 | 1 | 1 | 1 | -1 |
| 17 | 1 | 1 | -1 | 1 | -1 |
| 18 | 1 | -1 | 1 | 1 | -1 |
| 19 | -1 | 1 | -1 | 1 | 1 |
| 20 | 0 | 0 | 0 | 0 | 0 |

| **Value** | **MgSO₄** | **EtOH** | **TRAVASOL** | **rHSA** | **VES** |
|---|---|---|---|---|---|
| 0 | 0 mM | 0% | 0 mM | 0% | 0 mM |
| 1 | 30 mM | 3.07% | 4 mM | 0.5% | 2 mM |
| -1 | 15 mM | 1.54% | 1 mM | 0.05% | 1 mM |

PICOGREEN analysis showed that VES contributed about 30% of the impact and had a positive effect on the stability of the virus. Travasol contributed about 18% and had a negative impact. The other factors tested had a negligible impact in this analysis, both alone and in combination with other factors.

Considering both stability, as measured by PICOGREEN and analytical HPLC, and infectivity, as measured by hexon protein, the global ranking of the five excipients was VES > rHSA > ethanol > MgSO4 > TRAVASOL. VES had a positive impact on the adenovirus' ability to withstand heat treatment. VES accounted for 50% of the variability contribution to heat stability and 20% of the variability contribution to infectivity. Recombinant HSA increased the thermostability of the virus, while VES both increased the thermostability and prevented metal-catalyzed oxidation following light exposure. TRAVASOL and MgSO₄ both had a negative impact on infectivity, with the latter inducing a 17% loss in infectivity between days three and seven at 30°C.

### Example 2: Effect of Sugar Composition, Osmolality, VES and rHSA on Stability and Infectivity

ChAd155-RSV was formulated at a concentration of 1 x 10¹¹ PU/ml comprising a 10% overage in Tris pH 8.5, NaCl, polysorbate 80 (w/v), histidine, MgCl₂, trehalose, sucrose, rHSA and VES at concentrations shown in the table below for the first twelve compositions. Compositions 13-15 were formulated at 2x concentration to test the concept of a syrup and were then diluted prior to analysis.

| **#** | **Adenovirus** | **Tris pH 8.5 (mM)** | **Histidine (mM)** | **NaCl (mM)** | **MgCl₂ (mM)** | **Polysorbate 80 (%w/v)** | **Trehalose (%w/v)** | **Sucrose (%w/v)** | **rHSA (%w/v)** | **VES (mM)** | **Osmolality (mOsm)** |
|---|---|---|---|---|---|---|---|---|---|---|---|
| 1 | 1.1 x 10¹¹ | 10 | 10 | 5 | 1 | 0.020 | 7 (low) | 2 | 0 | 0 | 325 |
| 2 | 1.1 x 10¹¹ | 10 | 10 | 5 | 1 | 0.020 | 15 (mid) | 2 | 0 | 0 | 616 |
| 3 | 1.1 x 10¹¹ | 10 | 10 | 5 | 1 | 0.020 | 23(high) | 2 | 0 | 0 | 584 |
| 4 | 1.1 x 10¹¹ | 10 | 10 | 5 | 1 | 0.024 | 7 (low) | 2 | 0 | 0.5 | 333 |
| 5 | 1.1 x 10¹¹ | 10 | 10 | 5 | 1 | 0.024 | 15 (mid) | 2 | 0 | 0.5 | 640 |
| 6 | 1.1 x 10¹¹ | 10 | 10 | 5 | 1 | 0.024 | 23(high) | 2 | 0 | 0.5 | 1013 |
| 7 | 1.1 x 10¹¹ | 10 | 10 | 5 | 1 | 0.024 | 7 (low) | 2 | 0.1 | 0.5 | 337 |
| 8 | 1.1 x 10¹¹ | 10 | 10 | 5 | 1 | 0.024 | 15 (mid) | 2 | 0.1 | 0.5 | 645 |
| 9 | 1.1 x 10¹¹ | 10 | 10 | 5 | 1 | 0.024 | 23(high) | 2 | 0.1 | 0.5 | 1020 |
| 10 | 1.1 x 10¹¹ | 10 | 0 | 25 | 1 | 0.020 | 0 | 8 | 0 | 0 | 337 |
| 11 | 1.1 x 10¹¹ | 10 | 0 | 25 | 1 | 0.020 | 0 | 16 | 0 | 0 | 661 |
| 12 | 1.1 x 10¹¹ | 10 | 0 | 25 | 1 | 0.020 | 0 | 25 | 0 | 0 | 1151 |
| 13 | 2.2 x 10¹¹ | 20 | 20 | 7 | 2 | 0.040 | 35 | 2.2 | 0 | 0 | >1000 |
| 14 | 2.2 x 10¹¹ | 20 | 20 | 7 | 2 | 0.040 | 0 | 35 | 0 | 0 | >1000 |
| 15 | 2.2 x 10¹¹ | 20 | 20 | 7 | 2 | 0.040 | 0 | 48 | 0 | 0 | >1000 |

Each of the above compositions was tested for stability after being exposed to temperatures of 4°C for three months or 30°C for one month to examine the effects of high osmolality and sugar composition on stability and infectivity. Osmolality had little or no effect on either stability or infectivity.

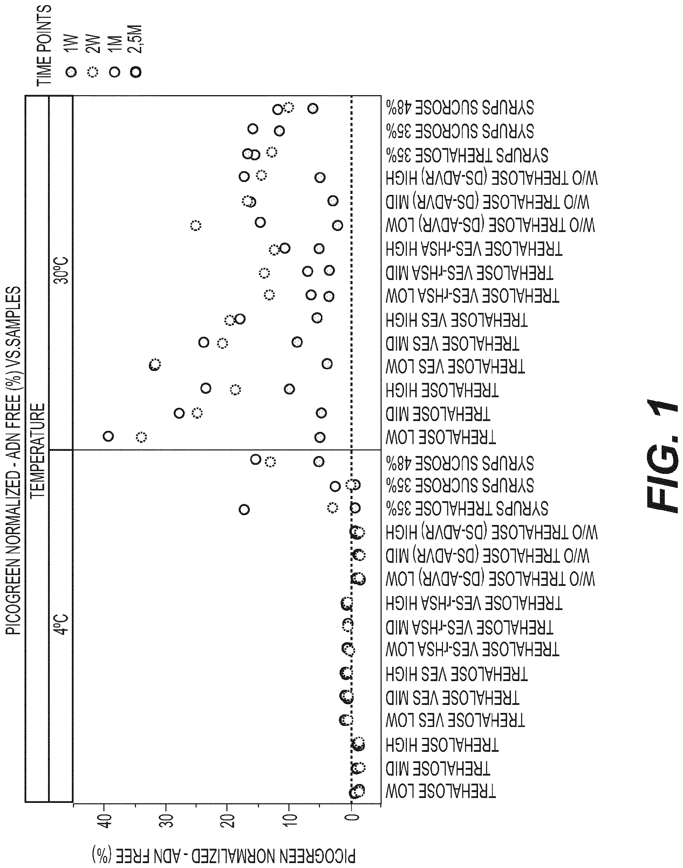

As shown in FIG. 1, most of the virus compositions were stable for at least three months at 4°C (left panel) and one week at 30°C (right panel). After three months at 4°C compositions 1-12 remained stable. After two weeks or one month at 30°C (right panel dotted line dots and dotted fill dots, respectively), the addition of VES to the compositions containing either low, medium or high concentrations of trehalose resulted in a decrease in the amount of free DNA, *i.e*., demonstrated increased viral stability compared to the composition in the absence of VES. The same positive effect on stability was observed with the addition of both VES and rHSA to compositions containing low, medium or high concentrations of trehalose. Replacing trehalose with sucrose reduced the beneficial effect of VES and VES + rHSA, as did doubling the sugar concentration to produce a syrup.

### Example 3: Effect of Trehalose, Sucrose, VES and rHSA on Stability

ChAd155-RSV was formulated as shown in the table below. Each formulation was tested for stability by analytical HPLC after being exposed to temperatures of 4°C or 25°C for one month to examine the effects of trehalose, sucrose, VES and rHSA on stability.

| | **Adenovirus (PU/ml)** | **Tris (mM)** | **Histidine (mM)** | **NaCl (mM)** | **MgCl2 (mM)** | **Polysorbate (%w/v)** | **Trehalose (%w/v**) | **Sucrose (%w/v)** | **rHSA ( %w/v )** | **VES (mM)** |
|---|---|---|---|---|---|---|---|---|---|---|
| 1 | 1.1 X 10¹¹ | 10 | 10 | 5 | 1 | 0.024 | 7 | 2 | 0 | 0 |
| 2 | 1.1 X 10¹¹ | 10 | 10 | 5 | 1 | 0.024 | 7 | 2 | 0 | 0.05 |
| 3 | 1.1 X 10¹¹ | 10 | 0 | 5 | 1 | 0.024 | 0 | 8 | 0 | 0 |
| 4 | 1.1 X 10¹¹ | 10 | 0 | 5 | 1 | 0.024 | 0 | 8 | 0 | 0.05 |
| 5 | 1.1 X 10¹¹ | 10 | 10 | 5 | 1 | 0.024 | 0 | 8 | 0.1 | 0.05 |
| 6 | 1.1 X 10¹¹ | 10 | 10 | 25 | 1 | 0.024 | 0 | 8 | 0 | 0 |
| 7 | 1.1 X 10¹¹ | 10 | 10 | 25 | 1 | 0.024 | 0 | 8 | 0.1 | 0.05 |

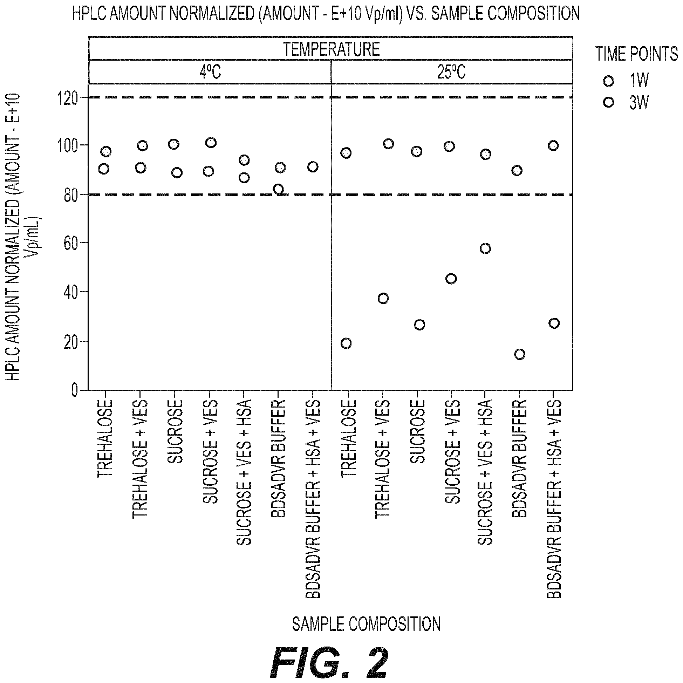

The virus was stable at 4°C for at least one month (not shown) and at 25°C for at least one week in each of the tested formulations (FIG 2). After three weeks at 25°C, some degradation began to occur and the differential effects of the formulation components were observed. VES increased viral stability when included in either sucrose-based or trehalose-based formulations. The addition of rHSA further increased stability, both in the presence and absence of added sugar.

### Example 4: Simian adenovirus stability after six months at 4°C

ChAd155-RSV was formulated in 10 mM Tris pH 8.5, 5 mM NaCl, 0.024% polysorbate 80 (w/v), histidine 10 mM and 1 mM MgCl₂. Sucrose (16%) and 0.05 mM VES was added to the buffer in the presence of 1.1 x 10¹¹ PU/ml, or 1.1 x 10¹⁰ PU/ml virus, taking into account a 10% overage. Trehalose (15%), 0.05 mM VES and 0.1% rHSA were added to the buffer at a concentration of 1.1 x 10¹¹ PU/ml or 1.1 x 10¹⁰ PU/ml virus, taking into account a 10% overage. Stability was tested at 4°C over the course of six months by sampling at days 0, 42, 91, 145 and 186 by HPLC in two separate HPLC runs.

| **Formulation:** | **Adenoviral Concentration (PU/ml)** | **Timepoint (days)** | **Stability Measured by HPLC (PU/ml)** | **Stability Measured by HPLC (PU/ml)** | **Infectivity Measured by IU (average) (IU/ml)** |
|---|---|---|---|---|---|
| 10 mM Tris pH 8.5, 10 mM histidine, | | | | | |
| 5 mM NaCl, 1 mM MgCl₂, 0.024% polysorbate 80 (w/v) | | | *Run 1* | *Run 2* | |
| with the addition of: | | | | | |
| | | 0 | 1.15 x 10¹¹ | 1.15 x 10¹¹ | ND |
| 15% trehalose | | 42 | 1.07 x 10¹¹ | 1.05 x 10¹¹ | |
| 2% sucrose | 1 x 10¹¹ | 91 | 1.02 x 10¹¹ | 1.00 x 10¹¹ | |
| 0.05mM VES | | 145 | 0.98 x 10¹¹ | 0.96 x 10¹¹ | |
| 0.1 % rHSA (w/v) | | 186 | 0.90 x 10¹¹ | 0.88 x 10¹¹ | |
| | | 0 | 1.19 x 10¹¹ | 1.10 x 10¹¹ | 9.39 x 10¹⁰ ± 0.14 |
| 16% sucrose | | 42 | 1.15 x 10¹¹ | 1.05 x 10¹¹ | 9.38 x 10¹⁰ ± 0.13 |
| 0.05mM VES | 1 x 10¹¹ | 91 | 1.14 x 10¹¹ | 1.04 x 10¹¹ | 9.38 x 10¹⁰ ± 0.14 |
| | | 145 | 1.12 x 10¹¹ | 1.03 x 10¹¹ | 9.33 x 10¹⁰ ± 0.22 |
| | | 186 | 1.10 x 10¹¹ | 1.01 x 10¹¹ | 9.26 x 10¹⁰ ± 0.16 |
| | | 0 | 1.08 x 10¹⁰ | 0.95 x 10¹⁰ | |
| 15% trehalose | 1 x 10¹⁰ | 42 | 1.06 x 10¹⁰ | 0.95 x 10¹⁰ | |
| 2% sucrose | | 91 | 1.07 x 10¹⁰ | 0.96 x 10¹⁰ | |
| 0.05mM VES | | 145 | 1.08 x 10¹⁰ | 0.93 x 10¹⁰ | |
| 0.1% rHSA | | 186 | 1.08 x 10¹⁰ | 0.95 x 10¹⁰ | |

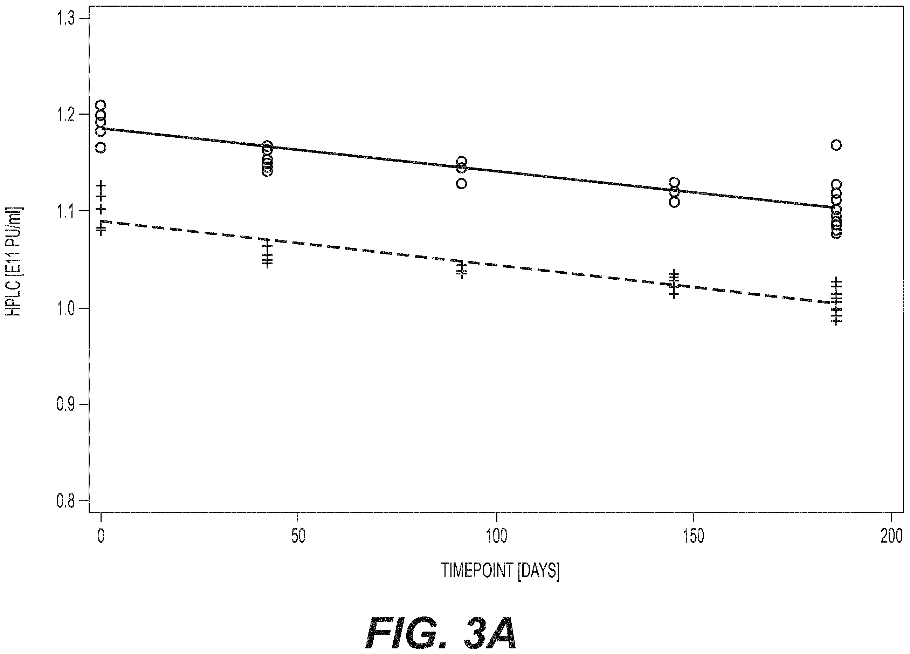

As shown in the table above and FIG. 3A, adenovirus formulated in 10 mM Tris pH 8.5, 5 mM NaCl, 0.024% polysorbate 80 (w/v), 1 mM MgCl₂, histidine 10 mM, 15% trehalose (w/v), 2% sucrose (w/v), 0.05 mM VES and 0.1% rHSA (w/v) at a concentration of 1.1 x 10¹¹ PU/ml observed over a period of 186 days at 4°C showed a decrease in stability. When extrapolated, these data project an average decline in total viral content of about 30% over two years and about 45% over three years. The adenovirus formulated in this manner also showed a decrease in infectivity, corresponding to an average decrease of about 70% over two years (IC95 = 14% - 90%) and about 83% in three years (IC95 = 20% - 97%).

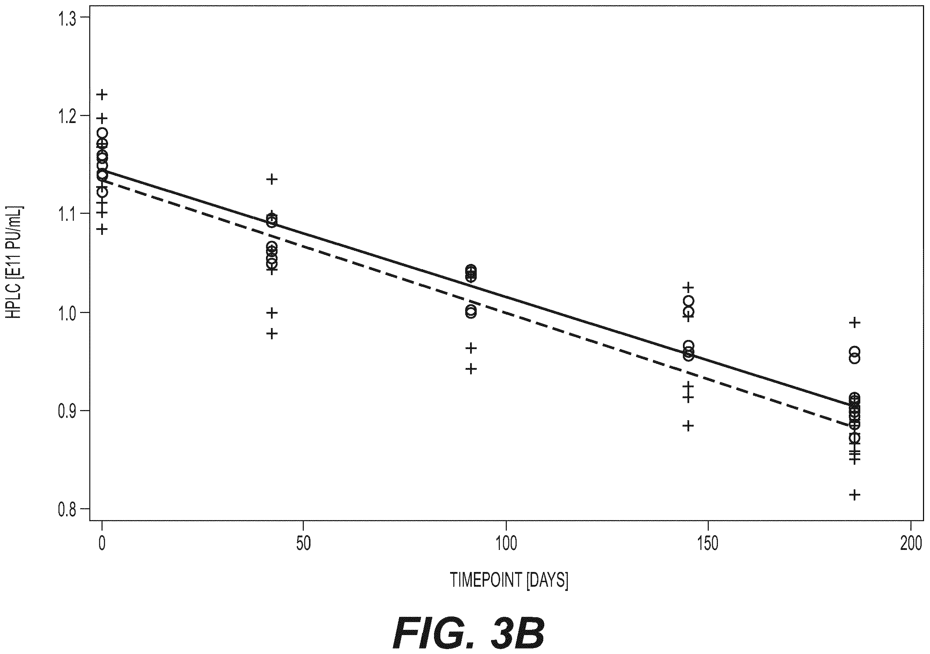

Adenovirus formulated in 10 mM Tris pH 8.5, 5 mM NaCl, 0.024% polysorbate 80 (w/v), 1 mM MgCl₂, histidine 10 mM, 16% sucrose, and 0.05 mM VES at a concentration of 1.1 x 10¹¹ PU/ml observed over a period of 186 days 4°C also showed a decrease in stability (Table and FIG. 3B). When extrapolated, these data project an average decline in total viral content of about 85% over two years.

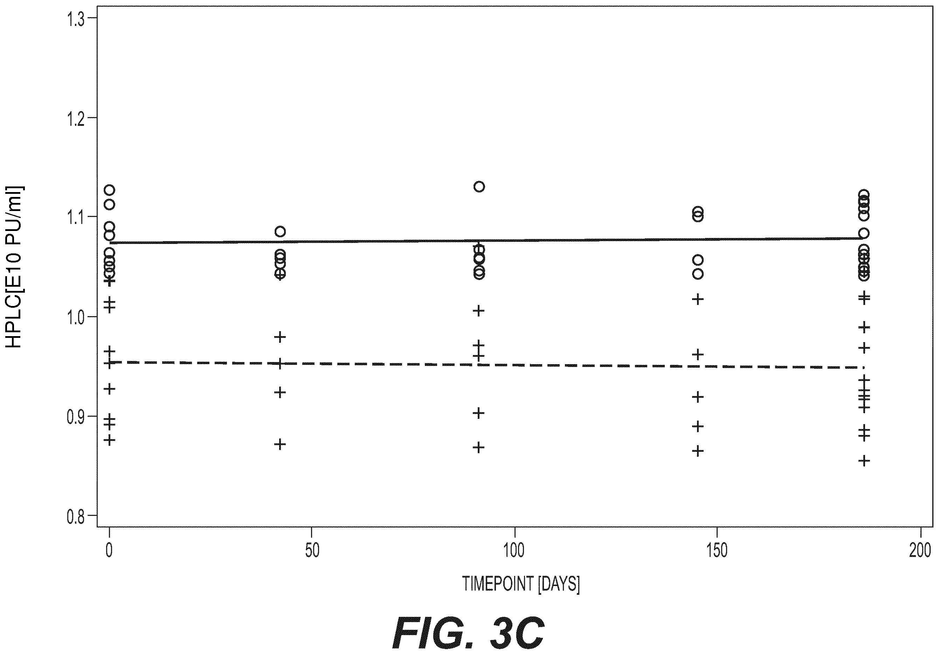

Adenovirus formulated in 10 mM Tris pH 8.5, 5 mM NaCl, 0.024% polysorbate 80 (w/v), 1 mM MgCl₂, histidine 10 mM, 15% trehalose (w/v), 2% sucrose (w/v), 0.05 mM VES and 0.1% rHSA (w/v) at a concentration of 1.1 x 10¹⁰ PU/ml observed over a period of 186 days 4°C showed no significant loss of stability (Table and FIG. 3C).

### Example 5: Accelerated Stability Evaluations

The two experiments shown below demonstrate that formulations of simian adenovirus comprising VES and rHSA are thermostable and retain their infectious properties.

### Experiment 1: 30 days at 4°C or 25°C

### Thermostability

Adenovirus was formulated at concentrations of 7.5x10¹⁰ PU/ml or 7.5x10⁹ PU/ml (including a 10% overage) in 10 mM Tris pH 8.5, 5 mM NaCl, 0.024% polysorbate 80 (w/v), 10 mM histidine and 1 mM MgCl₂, with the addition of either: (1) 16% sucrose (w/v) + 0.05 mM VES; (2) 16% sucrose (w/v) + 0.05% VES + 0. 1% rHSA (w/v); or (3) 15% trehalose (w/v) + 2% sucrose (w/v) + 0.05 mM VES + 0.1% rHSA (w/v). The virus was kept at a temperature of either 4°C or 25°C for 30 days. Stability was evaluated by analytical HPLC, PICOGREEN, and quantitative PCR in the presence and absence of benzonase, which is a nuclease that lacks photolytic activity.

| **Adenovirus Concentration (PU/ml)** | **Formulation** | **HPLC (PU/ml)** | | **PICOGREEN (%)** | | **qPCR (gE/ml) (+) or (-) benzonase** | |
|---|---|---|---|---|---|---|---|
| | 10 mM Tris pH 8.5, 10 mM histidine, 5 mM NaCl, 1 mM MgCl2, 0.024% polysorbate 80 (w/v) | **4°C** | **25°C** | **4°C** | **25°C** | **4°C** | **25°C** |
| | with the addition of: | | | | | | |
| | 16% sucrose | | | | | | |
| 7.5 x 10¹⁰ PU/ml | 0.05mM VES | 7.08 x 10¹⁰ | 5.70 x 10¹⁰ | 2.2% | 2.8% | (+) 6.29 x 10¹⁰ | (+) 5.30 x 10¹⁰ |
| | | | | | | (-) 6.69 x 10¹⁰ | (-) 5.71 x 10¹⁰ |
| | 16% sucrose | | | | | | |
| | 0.05mM VES | 7.40 x 10¹⁰ | 6.81 x 10¹⁰ | 2.6% | 3.8% | (+) 6.76 x 10¹⁰ | (+) 5.94 x 10¹⁰ |
| | 0.1% rHSA | | | | | (-) 6.83 x 10¹⁰ | (-) 7.02 x 10¹⁰ |
| | 15% trehalose | | | | | | |
| | 0.05mM VES | 7.33 x 10¹⁰ | 5.97 x 10¹⁰ | 2.6% | 7.1% | (+) 6.53 x 10¹⁰ | (+) 4.75 x 10¹⁰ |
| | 0.1% rHSA | | | | | (-) 7.09 x 10¹⁰ | (-) 6.48 x 10¹⁰ |
| 7.5 x 10⁹ PU/ml | 16% sucrose | | | | | | |
| | 0.05mM VES | 6.19 x 10⁹ | 6.15 x 10⁹ | | | | |
| | 16% sucrose | | | | | | |
| | 0.05mM VES | 6.13 x 10⁹ | 6.14 x 10⁹ | | | | |
| | 0.1% rHSA | | | | | | |
| | 15% trehalose | 6.37 x 10⁹ | 6.48 x 10⁹ | | | | |
| | 0.05mM VES | | | | | | |
| | 0.1% rHSA | | | | | | |

As shown in the table above, as measured by analytical HPLC, no significant degradation was observed after 30 days at 25°C in any of the three formulations tested at a viral concentration of 7.5 x 10⁹ PU/ml and there was no significant difference in stability among the formulations. At a viral concentration of 7.5 x 10¹⁰ PU/ml, a viral particle loss of 8-19% was observed, with the greatest loss occurring in the sucrose + VES formulation. Loss occurring in the sucrose + VES + rHSA formulation was significantly less than observed with the other two formulations.

As measured by PICOGREEN, at a viral concentration of 7.5 x 10¹⁰ PU/ml, the normalized average of DNA released from the viral capsid was higher after 30 days at 25°C than at 4°C with all three formulations.

As measured by qPCR, at a viral concentration of 7.5 x 10¹⁰ PU/ml, the number of genome equivalents per ml decreased in all three formulations after 30 days at 25°C, with the least decrease observed with sucrose + VES + rHSA in both the presence and absence of benzonase. These results demonstrate that all three compositions are thermostable simian adenovirus formulations.

### Infectivity

Adenovirus was formulated at a concentration of 7.5 x 10¹⁰ PU/ml in 10 mM Tris pH 8.5, 5 mM NaCl, 0.024% polysorbate 80 (w/v), histidine 10 mM , 1 mM MgCl₂, with the addition of either: (1) 16% sucrose (w/v) + 0.05 mM VES; (2) 16% sucrose (w/v) + 0.05mM VES + 0. 1% rHSA; or (3) 15% trehalose (w/v) + 0.05 mM VES + 0. 1% rHSA. The virus was kept at a temperature of either 4°C or 25°C for 30 days.

| **Adenovirus Concentration (PU/ml)** | **Formulation** | **Infectivity (log10(IU/ml))** | |
|---|---|---|---|
| | | **4°C** | **25°C** |
| | 16% sucrose | 8.88 | 7.92 |
| | 0.05mM VES | | |
| 6 x 10¹⁰ | 16% sucrose | 8.91 | 8.23 |
| | 0.05mM VES | | |
| | 0.1% rHSA | | |
| | 15% trehalose | 8.89 | 7.96 |
| | 0.05mM VES | | |
| | 0.1% rHSA | | |

Infectivity was determined by measuring infectious units by FACS analysis. At a viral concentration of 7.5 x 10¹⁰ PU/ml, some loss of infectivity was observed with all three formulations. The loss was significantly less in the sucrose + VES + rHSA than with the other two formulations. These results show that simian adenovirus retains infectivity when formulated in the above-described compositions.

### Experiment 2: 7 days at 30°C or 30 days at 25°C

### Stability

Adenovirus was formulated at a concentration of 7.5 x 10¹⁰ PU/ml in 10 mM Tris pH 8.5, 5 mM NaCl, 0.024% polysorbate 80 (w/v), histidine 10 mM,1 mM MgCl₂, with the addition of either: (1) 16% sucrose + 0.05 mM VES; (2) 16% sucrose + 0.05 mM VES + 0. 1% rHSA; or (3) 15% trehalose + 0.05 mM VES + 0.1% rHSA. The virus was either (1) stored at 4°C; kept at a temperature of 30°C for seven days; or kept at a temperature of 25°C for 30 days. Stability was evaluated by analytical HPLC, PICOGREEN, and quantitative PCR in the presence and absence of benzonase.

| **Adenovirus (PU/ml)** | **Formulation** | **HPLC (PU/ml)** | | | **PICOGREEN (%)** | | | **qPCR (gE/ml) (+) or (-) benzonase** | | |
|---|---|---|---|---|---|---|---|---|---|---|
| | | **4°C** | **7d 30°C** | **30d 25°C** | **4°C** | **7d 30°C** | **30d 25°C** | **4°C** | **7d 30°C** | **30d 25°C** |
| | 16% sucrose | | | | | | | | | |
| 6 x 10¹⁰ PU/ml | 0.05mM VES | 6.64 x 10¹⁰ | 6.00 x 10¹⁰ | 5.16 x 10¹⁰ | 1.8% | 2.5% | 2.7% | (+) 6.54 x 10¹⁰ | (+) 6.00 x 10¹⁰ | (+) 5.28 x 10¹⁰ |
| | | | | | | | | (-) 6.62 x 10¹⁰ | (-) 6.08 x 10¹⁰ | (-) 5.45 x 10¹⁰ |
| | 16% sucrose | | | | | | | | | |
| | 0.05mM VES | 6.83 x 10¹⁰ | 6.54 x 10¹⁰ | 6.23 x 10¹⁰ | 1.8% | 2.8% | 3.8% | (+) 7.06 x 10¹⁰ | (+) 6.91 x 10¹⁰ | (+) 6.57 x 10¹⁰ |
| | 0.1% rHSA | | | | | | | (-) 6.75 x 10¹⁰ | (-) 6.51 x 10¹⁰ | (-) 6.87 x 10¹⁰ |
| | 15% trehalose | | | | | | | | | |
| | 0.05mM VES | 6.79 x 10¹⁰ | 6.21 x 10¹⁰ | 5.24 x 10¹⁰ | 2.6% | 4.1% | 6.4% | (+) 6.66 x 10¹⁰ | (+) 6.15 x 10¹⁰ | (+) 5.15 x 10¹⁰ |
| | 0.1% rHSA | | | | | | | (-) 6.67 x 10¹⁰ | (-) 6.44 x 10¹⁰ | (-) 6.83 x 10¹⁰ |

As shown in the table above, as measured by analytical HPLC, a significant viral particle loss occurred in all three formulations under both accelerated stability conditions. The loss was significant after seven days at 30°C, with a viral particle loss of 4-10% observed. Viral particle loss in sucrose + VES + rHSA was significantly less than with the other two formulations.

As measured by PICOGREEN, the normalized average of DNA released from the viral capsid was higher after seven days at 30°C and significantly higher after 30 days at 25°C than at 4°C in all three formulations. trehalose + VES + rHSA showed a significantly greater decrease in stability than the other two formulations after 30 days at 25°C. The percentage of released DNA was based on a regression between a freshly thawed control group and a control degraded by exposure to 30 min. at 60°C, which completely degraded the viral capsid.

As measured by qPCR, the number of genome equivalents per ml significantly decreased after 30 days at 25°C (benzo (+)) in all three formulations and after seven days at 30°C in sucrose + VES and trehalose + VES + rHSA. Again, while the best results were obtained with sucrose + VES + rHSA, all three formulations supported an acceptable thermostability profile.

### Infectivity

Adenovirus was formulated at a concentration of 7.5 x 10¹⁰ PU/ml in 10 mM Tris pH 8.5, 5 mM NaCl, 0.02'% polysorbate 80 (w/v), histidine 10 mM ,1 mM MgCl₂, with the addition of either: (1) 16% sucrose (w/v) + 0.05 mM VES; (2) 16% sucrose (w/v) + 0.05mM VES + 0. 1% rHSA (w/v); or (3) 15% trehalose (w/v) + 0.05 mM VES + 0. 1% rHSA (w/v). The virus was either stored at 4°C or kept at a temperature of 30°C for seven days or 25°C for 30 days. Infectivity was determined by measuring infectious units by FACS analysis.

| **Adenovirus Concentration (PU/ml)** | **Formulation** | **Infectivity Measured by IU [log(IU/mL)]** | | | **Infectivity Ratio** | | |
|---|---|---|---|---|---|---|---|
| | | **4°C** | **7d** | **30d** | **4°C** | **7d** | **30d** |
| | | | **30°C** | **25°C** | | **30°C** | **25°C** |
| 6 x 10¹⁰ PU/ml | 16% sucrose 0.05mM VES | 9.03 | 8.72 | 8.21 | 47 | 114 | 319 |
| | 16% sucrose 0.05mM VES 0.1% rHSA | 9.07 | 8.76 | 8.44 | 58 | 114 | 224 |
| | 15% trehalose 0.05mM VES 0.1% rHSA | 9.07 | 8.76 | 8.25 | 58 | 107 | 293 |

Some loss of viral infectivity was observed in all three formulations after seven days at 30°C and after 30 days at 25°C. A significantly smaller decrease was observed with the sucrose + VES + rHSA formulation than with the other two formulations after 30 days at 25°C. The infectivity ratio, i.e., the ratio of the number of infectious viral particles to the number of total viral particles, was determined by calculating the ratio of analytical HPLC to IU results. A significant increase in the infectivity ratio was observed with all three formulations after seven days at 30°C and after 30 days at 25°C. The smallest increase was observed with the sucrose + VES + rHSA formulation after 30 days at 25°C.

### Example 6: Extrapolation of Stability Data

Data from an extrapolation of stability study were analyzed to estimate a loss of viral particle stability over time with statistical models that extrapolate viral particle loss over time. Both linear and first-order decay models were used and the results shown in the table below.

| | **Formulation** | **Adenoviral Concentration (PU/ml)** | **Observed Viral Particle Loss** | **Viral Particle Loss extrapolated to two years** | |
|---|---|---|---|---|---|
| | 10 mM Tris pH 8.5, (mM NaCl, 0.02% polysorbate 80 (w/v) 1 mM MgCl₂. with the addition of : | | | **Linear model (95% Cl)** | **First-order decay model (95% Cl)** |
| **Viral Particle Stability (HPLC)** | *Trehalose 15% VES 0.05mM rHSA 0.1%* | 6 x 10¹⁰ | -4% (six months) | -15% (-24%; -5%) | -7% (-22%; -5%) |
| | | 6 x 10⁹ | -1% (six months) | -2% (*-6%;* +*2*%*)* | -*2*% *(-7*%; +*3%)* |
| | *Sucrose 16% VES 0.05mM rHSA 0.1%* | 6 x 10¹⁰ | -1% (10 months) | -2% (-4%; 0%) | -2% (-3%; 0%) |
| | | 6 x 10⁹ | *No loss observed* | *3% (-4%;* +*11%)* | *3% (-5%;* +*12%)* |

| **Infectivity (Protein M)** | ***Formulation*** | ***Adenoviral Concentration (PU*/*ml)*** | ***Observed Infectivity Decline*** | ***Infectivity Decline extrapolated to two years*** | |
|---|---|---|---|---|---|
| | | | | **Linear Model (95% CI)** | **First-order decay model (95% CI)** |
| | *Trehalose 15% VES 0.05mM rHSA 0.1%* | 6x10¹⁰ | -15% (six months) | -62% (-85%; -38%) | -49% (-60%; - 33%) |
| | | 6x10⁹ | -15% (six months) | -58% (-77%; -40%) | -47% (-57%; - 34%) |
| | *Sucrose 16% VES 0.05mM rHSA 0.1%* | 6x10¹⁰ | -35% (10 months) | -52% (-65%; -39%) | -45% (-53%; - 36%) |
| | | 6x10⁹ | -10% (six months) | -31% (-49%; -13%) | -27% (-40%; - 13%) |

Italics indicate that the estimated loss is not statistically significant. Adenovirus at concentrations below 6 x 10¹⁰ PU/ml showed little or no decrease in stability over three years at 4°C, compared to that observed at the higher concentrations.

### Example 7: Immunogenicity

Balb/c mice were immunized with either 3 x 10⁷ or 3 x 10⁶ viral particles of ChAd155-RSV formulated in (a) 10 mM Tris pH 8.5, 10 mM histidine, 5 mM NaCl, 16% sucrose (w/v), 0.024% polysorbate 80 (w/v), 1 mM MgCl₂, 0.05 mM VES and 0.1% rHSA or (b) the lyophilized formulation of WO 2017/013169).

T-cell response was measured three weeks after the immunization by *ex vivo* IFN-gamma enzyme-linked immunospot (ELISpot) using the RSV-M2 immunodominant CD8 epitope. Each dot represents the response in a single mouse.. Results are expressed as IFN gamma spot forming cells per million splenocytes. Horizontal lines represent the mean number of IFN-gamma SFC/million splenocytes for each dose group "geomean."

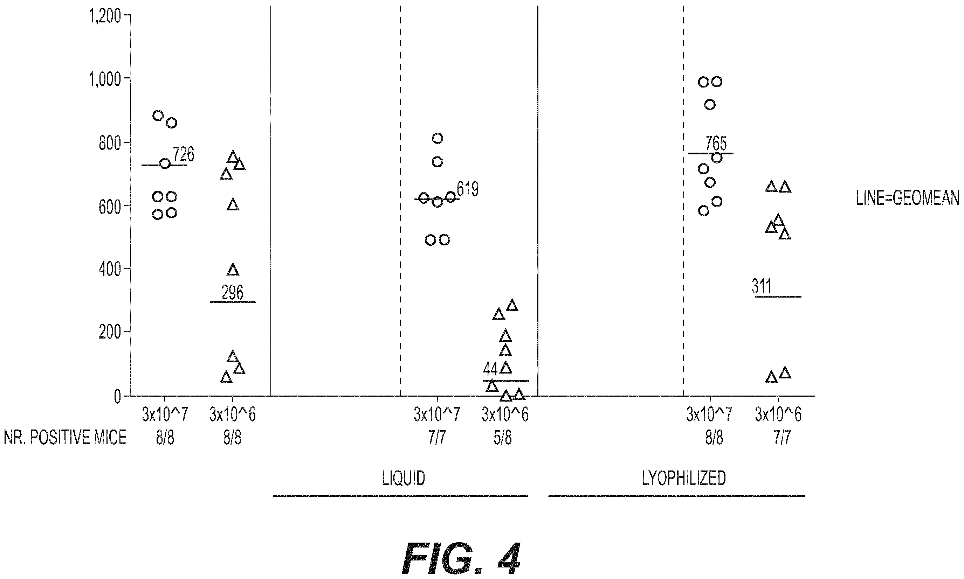

FIG. 4 demonstrates that RSV-specific T cells were detected in the spleens of the mice immunized with either 3 x 10⁷ or 3 x 10⁶ viral particles of ChAd155-RSV. The liquid composition of the invention compared favorably with the lyophilized formulation. Therefore, compositions of the invention can be used as immunogenic simian adenoviral formulations.

### Example 8: Forced Degradation by Exposure to Metal and Light Oxidation

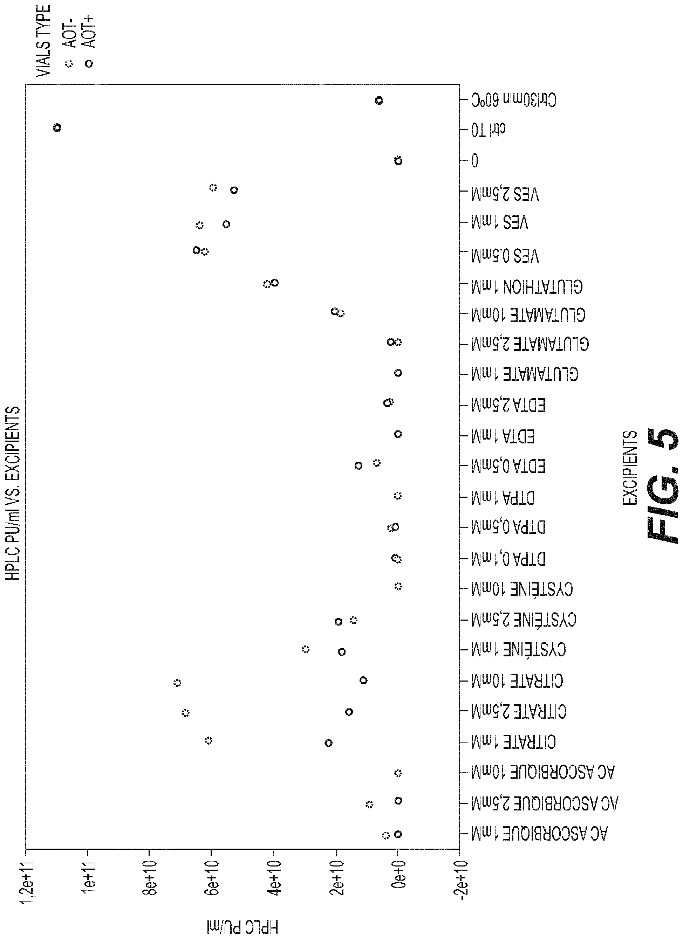

ChAd155-RSV was formulated at a concentration of 3 x 10¹⁰ or 2.8 x 10¹¹ viral particles per milliliter in 10 mM Tris pH 8.5, 25 mM NaCl, 8% sucrose (w/v), 0.02% polysorbate 80 (w/v) and 1 mM MgCl₂, then exposed to metal and light oxidation under Accelerated Oxidation Test (AOT) conditions for three, eight or ten days at 30°C, 37°C or 42°C. Light irradiation conditions were 765 W/m² at 320-380 nm. The oxidation conditions exposure adenovirus formulations to a metal cocktail comprising 1.5 ppm each of Cu⁺², Ni⁺², Fe⁺³ and Cr⁺³ under the above light irradiation conditions. Excipients tested included ascorbic acid, citrate, cysteine, diethylenetriaminepentaacetic acid (DTPA), ethylenediaminetetraacetic acid (EDTA), glutamate and vitamin E succinate (VES). Stability was measured by analytical high performance liquid chromatography (HPLC) and the results were expressed as HPLC virus particle units per milliliter (PU/ml). Dotted line dots indicate stability in the absence of the AOT conditions (AOT (-) vials) and solid line dots indicate stability in the presence of the AOT conditions (AOT (+) vials. As shown in FIG. 5, the best results were obtained with VES; stability remained high in both the presence and absence of metal and light-induced oxidation.

## Claims

1. An aqueous composition comprising a simian adenoviral vector; a buffer selected from Tris, borate and succinate; histidine; NaCl; a bivalent metal salt selected from MgCl₂, CaCl₂ and MgSO₄; a surfactant selected from a poloxamer surfactant and a polysorbate surfactant; an alpha tocopherol succinate, recombinant human serum albumin and sucrose.

2. The aqueous composition of claim 1, further comprising trehalose.

3. The aqueous composition of claim 1 or claim 2, wherein the buffer is about 1.0 to 25 mM Tris pH 6-10.

4. The aqueous composition of any of the preceding claims, wherein the concentration of histidine is less than about 15 mM.

5. The aqueous composition of any of the preceding claims, wherein the concentration of NaCl is less than about 30 mM.

6. The aqueous composition of any of the preceding claims, wherein the bivalent metal salt is MgCl₂.

7. The aqueous composition of any of the preceding claims, wherein the surfactant is polysorbate 80.

8. The aqueous composition of any of the preceding claims, wherein the alpha tocopherol succinate is Vitamin E succinate.

9. The aqueous composition of any of the preceding claims, wherein the concentration of human serum albumin is less than about 1% (w/v).

10. The aqueous composition of any of the preceding claims, wherein the concentration of sucrose is less than about 30% (w/v), for example, less than about 25% (w/v), about 10% to about 20% (w/v) or about 16% (w/v).

11. The aqueous composition of any of claims 2-10, wherein the concentration of trehalose is about 1% to about 30% (w/v), for example, about 10% to about 20% (w/v), about 15% to about 16% (w/v).

12. The aqueous composition of claim 1, wherein the simian adenoviral vector is a chimpanzee, bonobo or gorilla vector.

13. The aqueous composition of claim 12, wherein the simian adenoviral vector is a chimpanzee vector, for example, a serotype C chimpanzee vector or a chimpanzee adenoviral vector selected from ChAd3, ChAd63, ChAd83, ChAd155 and ChAd157.

14. The aqueous composition of any of the preceding claims, wherein the concentration of the simian adenoviral vector is between about 1 x 10⁶ and 1 x 10¹² viral particles per milliliter.

15. The aqueous composition of any of the preceding claims, wherein the aqueous composition comprises a transgene, for example, an immunogenic transgene.

16. The aqueous composition of any of the preceding claims, wherein the simian adenoviral vector is replication defective or wherein the simian adenoviral vector is replication competent.

17. A method of making an aqueous liquid composition according to any of the preceding claims comprising
a. producing a simian adenovirus in a host cell; and
b. combining the adenovirus with one or more excipients;
wherein the adenovirus is stable (i) at +2 - +8°C for at least two years or (ii) at 25°C for at least 30 days or (iii) at 30°C for at least seven days.

18. The composition of any of claims 1-16 for use as a vaccine.
